Europäisches Patentamt

European Patent Office

Office européen des brevets

(11) Publication number: **0 181 790**
**B1**

(12) **EUROPEAN PATENT SPECIFICATION**

(45) Date of publication of patent specification: **01.08.90**

(21) Application number: **85401921.3**

(22) Date of filing: **02.10.85**

(51) Int. Cl.⁵: **C 07 C 25/02,** C 07 C 17/156, B 01 J 29/28, B 01 J 29/34

(54) **Method for the synthesis of iodobenzene.**

(30) Priority: **05.10.84 IT 2300484**

(43) Date of publication of application:
**21.05.86 Bulletin 86/21**

(45) Publication of the grant of the patent:
**01.08.90 Bulletin 90/31**

(84) Designated Contracting States:
**BE DE FR GB IT NL**

(56) References cited:

CHEMICAL ABSTRACTS, vol. 102, no. 19, 13 May 1985, Columbus, OH (US); p. 590, no. 166447a

(73) Proprietor: **MONTEDIPE S.r.l.**
**31, Foro Buonaparte**
**I-20121 Milan (IT)**

(72) Inventor: **Paparatto, Giuseppe**
**7 via Vasari Cinisello Balsamo**
**Milan (IT)**
Inventor: **Saetti, Marco**
**73 via Pentapoli**
**I-96010 Priolo Siracusa (IT)**

(74) Representative: **Hirsch, Marc-Roger**
**Cabinet Hirsch 34 rue de Bassano**
**F-75008 Paris (FR)**

Courier Press, Leamington Spa, England.

**Description**

The present invention concerns a process for the synthesis of iodobenzene by oxidative iodination, in the gaseous phase, of benzene with iodine and oxygen, air or another oxygen containing gas.

The synthesis of iodobenzene starting from benzene and iodine is usually carried out in the liquid phase, in the presence of an oxidative agent; preferably the oxidative agent consists of nitric acid (see Japanese patent 58/77830, U.S.S.R. patent 453392 and the article of Datta, R. L. and Chatterjee N. R.: J. Am. Chem. Soc. 39, 437, 1917). Other oxidative agents may also be used; none of which, however, has proved, hitherto, to be more efficient and convenient than nitric acid. For instance, the use of iodic acid, sulfur trioxide and hydrogen peroxide has been disclosed (see Butler A. R.: J. Chem. Educ. 36, 508, 1971) as well as the aromatic iodination, catalyzed by metal halogenides, (see Uemura S., Onoe A., Okano M, Bull. Chem. Soc. Jpn. 47, 147, 1974).

Recently, Italian patent application 19860 A/84, in the name of the Applicant, has described a process for the synthesis of aromatic iodo-substituted compounds starting from alkaline salts of aromatic acids, by reaction with iodine. In all the well known methods, the selectivities, with respect to iodine, have never been higher than 90%; in certain methods expensive oxidative agents are utilized and in other methods the starting products comprise reactants that are readily available.

Now, it has been observed that the synthesis of iodobenzene can be carried out more conveniently according to the reaction:

$$2C_6H_6 + I_2 + 1/2O_2 \rightarrow 2C_2H_5I + H_2O \qquad (I)$$

using $O_2$ as oxidative agent and a particular zeolite as catalyst.

In its widest form, the invention relates to a method for the synthesis of iodobenzene in a gaseous phase, by iodination of benzene with iodine, in the presence of oxygen, air or another oxygen containing gas, in the presence of a zeolitic catalyst, selected from the group comprising the ZSM—5 and ZSM—11 types zeolites, exchanged, before being used, with at least one bi-valent or trivalent metal cation. More specifically, the bivalent or trivalent cation, is preferably selected from the group comprising the $Mn^{++}$, $Co^{++}$, $Zn^{++}$, $Fe^{++}$ and $Ni^{++}$ cations, as well as the alkaline-earth metal cations.

The zeolitic catalyst may be used as such or mixed with a suitable amount of an inert agent, for instance $SiO_2$, acting as a binder and/or a carrier.

When a zeolite, according to the invention, is used as catalyst, a stabilization of the catalytic activity is noted. When, on the contrary, the same zeolites are used in the acid or alkaline form, a deterioration decay of said activity is noted. Furthermore, in the case of zeolites having a low $SiO_2/Al_2O_3$ ($\leqq 10$) ratio, the acid form can be in no case be used, since a structure collapse takes place under the reaction conditions or during the possible reactivation.

For the catalyst preparation it is possible to start from both the sodic and acidic form. In the first case the sodium is exchanged with the desired cation, using known methods; in the second case, where the starting zeolites are the acidic form, although the exchange technique can still be used, it is more convenient to utilize the neutralization technique, using an aqueous solution of a salt of a cation which by hydrolysis gives a basic pH or preferably a diluted solution of the hydroxide of a metal cation. This later method ensures obtention of a zeolite free from Brönsted acid sites. The catalytic system can also consist of zeolites, having two or more metal cations.

The iodination can be carried out according to any one of the different known techniques, without departing from the spirit and scope of the invention. By way of example, however, general operating directions are preferred for carrying out the process. An iodine solution in benzene (concentration from 0.5 to 50% and preferably from 5 to 20% by weight) is evaporated and mixed with air in an amount such that the air/$I_2$ molar ratio is at least equal to the stoichiometric ratio and preferably $\geqq 10$. The resultant mixture is conveyed to a fixed bed reactor, loaded with the catalyst, the temperature being comprised between 200 and 550°C (preferably between 350 and 450°C) and the space velocity (WHSV) comprised between 0.1 and 100 kg/h (preferably between 1 and 20 kg/h) of benzene per kg of the active part of the catalyst (binder excluded). It is also possible to utilize an inert diluent, such as, for instance, nitrogen, helium or steam. The products can then be recovered by cooling the gaseous flow leaving the reactor and by resorting to conventional treatments. In case of distillation, the overhead distilled benzene can be recycled to the iodination reactor. The overall pressure used during the tests is almost always slightly higher than the atmospheric pressure; lower or higher pressures, however, may also be used. The catalyst maintains its activity for a long period of time, particularly when working in a gas phase at 300—345°C. However, once the catalytic activity falls below the allowable levels, regeneration is required. An excellent regeneration consists in activating the catalyst in benzene-air mixtures for a few hours at temperatures comprised between 300 and 600°C. The starting activation of the catalyst is also an important element.

The following examples will illustrate the invention, without limiting, however, the scope thereof.

Example 1
(comparative: H—ZSM—5)

A ZSM—5 zeolite was prepared in a raw form, according to example 24 of U.S. patent 3702886 and was

2

then exchanged at 80°C with a 1M solution of HCl in order to obtain the H—ZSM—5 form free from sodium; the zeoloite crystallites had an average size lower than 0,5 µm.

1 g of the H—ZSM—5 zeolite thus obtained was mixed with 0,3 g of binder ($SiO_2$) and said mixture was activated in air for 2 hours at 540°C. The resultant catalyst was loaded into a quartz microreactor, maintained at 400°C by means of a thermostat and continuously fed with a gaseous mixture of benzene, iodine and air, having a benzene/iodine/air molar ratio = 20/1/20. The pressure was slightly higher than 760 mm/Hg and the space velocity (WHSV) was 6 kg/h of benzene/iodine mixture per kg of zeolite. The reaction was run for 1 hour and the reaction products were recovered by condensation. The iodine conversion was 65% and the molar selectivity to iodobenzene (with respect to benzene) 98.6%; after 6 hours the conversion dropped to about 25%.

## Example 2
### (comparative: Na—ZSM—5

1 g of H—ZSM—5 zeolite, prepared as described in example 1 and supported by 0.3 g of $SiO_2$, was suspended in 20 cm$^3$ of deionized water and neutralized with a 0.1M solution of NaOH; the suspension (at pH 8) was heated to 80°C. In case where the pH has a tendency to drop, NaOH was added to maintain a persistent pH 8. The suspension was filtered and the solid was washed with deionized water, dried in an oven at 110°C for 2 hours and activated at 540°C for a further 2 hours. The operating conditions of example 1 were then repeated, using the Na—ZSM—5 zeolite thus obtained; after 2 hours the iodine conversion was not higher than 20%.

## Example 3
### (Ca—ZSM—5)

Example 2 was repeated, while replacing the NaOH solution with an equivalent Ca(OH)$_2$ solution, using for the iodination the Ca—ZSM—5 zeolite thus obtained; data and results are set forth in Table 1.

## Example 4
### (Zn—ZSM—5)

1 g of H—ZSM—5 zeolite prepared as described in Example 1 and bound with 0.3 g of $SiO_2$, was exchanged three times at 80°C with 20 cm$^3$ (at a time) of a 0.5M solution of zinc acetate; when the exchange was completed the zeolite was washed with deionized water, dried at 110°C for 2 hours and activated at 540°C for a further 2 hours. Example 1 was then repeated by using the Zn—ZSM—5 zeolite thus prepared as catalyst; data and results are set forth in Table 1.

## Example 5
### (Co—ZSM—5)

Example 4 was repeated, while using a saturated solution of Co$^{++}$ acetate instead of zinc acetate; data and results are set forth in Table 1.

## Example 6

Example 5 was repeated, while lowering the reaction temperature to 350°C; data and results are set forth in Table 1.

## Example 7

Example 4 was repeated, while using a solution of Mn$^{++}$ acetate, instead of zinc acetate; data and results obtained are set forth in Table 1.

## Examples 8—13

Examples 3—7 were repeated, while varying slightly the operating parameters (flow and/or temperature); data and results obtained are set forth in Table 1.

## Example 14
### (Ba—ZSM—5)

Example 4 was repeated, while replacing the solution of zinc acetate with a solution of barium hydroxide; data and results obtained are set forth in Table 1.

## Example 15
### (comparative K—ZSM—5)

Example 4 was repeated, while replacing Ba(OH)$_2$ with potassium hydroxide; data and results obtained are set forth in Table 1.

## Example 16
### (Al—ZSM—5)

Example 14 was repeated replacing Ba(OH)$_2$ with aluminium hydroxide; data and results obtained are set forth in Table 1.

TABLE 1

| Ex. | Zeolite | T (°C) | WHSV (h$^{-1}$) | Conv. $I_2$ (%) | | Selectivity (%) | | | | | |
|---|---|---|---|---|---|---|---|---|---|---|---|
| | | | | | | $C_6H_5 I$ | | $C_6H_4 I_2$ | | others | |
| | | | | 1 h | 6 h | 1 h | 6 h | 1 h | 6 h | 1 h | 6 h |
| 1* | H-ZSM5 | 400 | 6 | 65 | 25 | 98.6 | n.d. | - | - | - | - |
| 2* | Na-ZSM5 | 400 | 6 | - | 20(2h) | - | - | - | - | - | - |
| 3 | Ca-ZSM5 | 400 | 6 | - | 99.5 | - | 97.5 | - | 2.2 | - | 0.3 |
| 4 | Zn-ZSM5 | 400 | 6 | - | 90.0 | - | 98.6 | - | 1.2 | - | 0.2 |
| 5 | Co-ZSM5 | 400 | 6 | - | 99 | - | 98.5 | - | 1.3 | - | 0.2 |
| 6 | Co-ZSM5 | 350 | 6 | - | 98.6 | - | 98.5 | - | 1.4 | - | 0.1 |
| 7 | Mn-ZSM5 | 400 | 6 | - | 99.6 | - | 97.8 | - | 2.1 | - | 0.1 |
| 8 | Ca-ZSM5 | 400 | 5.7 | 100 | 99.0 | 97.0 | 98.0 | 2.8 | 1.8 | 0.2 | 0.2 |
| 9 | Ca-ZSM5 | 350 | 5.7 | 99 | 78 | 98.0 | 98.5 | 1.9 | 1.5 | 0.1 | - |
| 10 | Mn-ZSM5 | 400 | 5.7 | 99.0 | 99.4 | 97.4 | 97.6 | 2.2 | 2.3 | 0.5 | 0.2 |
| 11 | Mn-ZSM5 | 350 | 5.7 | 96.3 | 80.0 | 98.3 | 98.8 | 1.5 | 1.0 | 0.2 | 0.2 |
| 12 | Zn-ZSM5 | 400 | 5.7 | 81.0 | 91.7 | 96.3 | 98.3 | 1.4 | 1.1 | 2.3 | 0.6 |
| 13 | Co-ZSM5 | 400 | 5.7 | 99.7 | 99.0 | 98.6 | 98.4 | 1.3 | 1.6 | 0.1 | - |
| 14 | Ba-ZSM5 | 400 | 5.7 | 82 | 75 | 98.6 | 98.5 | 1.4 | 1.5 | - | - |
| 15* | K-ZSM5 | 400 | 5.7 | 5 | - | 98 | - | 2.0 | - | - | - |
| 16 | Al-ZSM5 | 400 | 5.7 | 87 | 50 | 98.4 | 97.9 | 1.4 | 1.9 | 0.2 | 0.2 |

* Comparative.

EP 0 181 790 B1

Example 17
(Mg—ZSM—5)
Example 14 was repeated replacing Ba(OH)₂ with magnesium acetate; similar results were obtained.

Example 18
(Mg—ZSM—11)
Example 17 was repeated replacing the ZSM—5 zeolite with a ZSM—11 zeolite, thereby obtaining substantially similar results.

Example 19
(Mg—Mn—ZSM—5)
Example 7 was repeated, while using a 50/50 mixture (by moles) of $Mn^{++}$ acetate and Mg acetate; the results obtained were slightly better compared with results of both Example 7 and Example 17.

**Claims**

1. A method for the synthesis of iodobenzene by oxidative iodination, in the gaseous phase, of benzene with iodine and oxygen, or air or another oxygen containing gas, in the presence of a zeolitic catalyst, selected from the group comprising the zeolites of ZSM—5 and ZSM—11 type, exchanged, before being used, with at least one bi-valent or trivalent metal cation.

2. A method according to claim 1, characterized in that the iodination temperature is comprised between 200 and 550°C.

3. A method according to claim 2, characterized in that the iodination temperature is comprised between 350 and 450°C.

4. A method according to claim 1, characterized in that the oxidizing agent is air and the air/$I_2$ molar ratio is equal to or higher than the stoichiometric ratio.

5. A method according to claim 3, characterized in that the said molar ratio is higher than 10.

6. A method according to claim 1, characterized in that the concentration of iodine in the feed benzene is comprised between 0.5 and 50%.

7. A method according to claim 6, characterized in that the concentration of iodine in the feed benzene is comprised between 5 and 20% by weight.

8. A method according to claim 1, characterized in that the space velocity is comprised between 0.1 and 100 of benzene per kg of pure zeolite (binder excluded).

9. A method according to claim 8, characterized in that the the space velocity is comprised between 1 and 20 kg/h of benzene per kg of pure zeolite (binder excluded).

10. A method according to claim 6, characterized in that the zeolite is exchanged, prior to being used, with at least one cation selected from the group comprising $Zn^{++}$, $Co^{++}$, $Mn^{++}$ and the alkaline-earth cations.

11. A method for the synthesis of iodobenzene, characterized in that benzene, iodine and air (or another oxygen containing oxygen) are brought into contact with a zeolite of ZSM—5 type, exchanged with at least one cation selected from the group comprising $Zn^{++}$, $Mn^{++}$ and $Ca^{++}$, at temperatures comprises between 350°C and 450°C and with air/$I_2$ molar ratios equal to or higher than 8, the space velocity being comprised between 1 and 100 kg/h of benzene per kg of pure zeolite and iodine being fed as a benzenic solution at 5—20% b.w.

**Patentansprüche**

1. Verfahren zur synthetischen Herstellung von Jodbenzol durch oxydierende Jodierung von Benzol in gasförmiger Phase mit Hilfe von Jod und Sauerstoff, Luft oder einem anderen sauerstoffhaltigen Gas in Gegenwart eines zeolitischen Katalysators aus der Gruppe der ZSM—5 und ZSM—11 Zeoliten mit vor der Anwendung erfolgendem Austausch von wenigstens einem zwei- oder dreiwertigen Metallkation.

2. Verfahren nach Anspruch 1, dadurch gekennzeichnet, dass die Jodierungstemperatur zwischen 200 und 550°C liegt.

3. Verfahren nach Anspruch 2, dadurch gekennzeichnet, dass die Jodierungstemperatur zwischen 350 und 450°C liegt.

4. Verfahren nach Anspruch 1, dadurch gekennzeichnet, dass als Oxydationsmittel Luft verwendet wird und dass das Molekularverhältnis Luft/$I_2$ mindestens gleich gross oder grösser als das stoechiometrische Verhältnis ist.

5. Verfahren nach Anspruch 3, dadurch gekennzeichnet, dass das Molekularverhältnis grösser als 10 ist.

6. Verfahren nach Anspruch 1, dadurch gekennzeichnet, dass die Jodkonzentration im zugeführten Benzol zwischen 0,5 und 50 Gew./% liegt.

7. Verfahren nach Anspruch 6, dadurch gekennzeichnet, dass die Jodkonzentration im zugeführten Benzol zwischen 5 und 20 Gew./% liegt.

8. Verfahren nach Anspruch 1, dadurch gekennzeichnet, dass die Raumgeschwindigkeit zwischen 0,1 und 100 kg/h Benzol pro kg Reinzeolit (Binder ausgeschlossen) liegt.

9. Verfahren nach Anspruch 8, dadurch gekennzeichnet, dass die Raumgeschwindigkeit zwischen 1 und 20 kg/h Benzol pro kg Reinzeolit (Binder ausgeschlossen) liegt.

10. Verfahren nach Anspruch 6, dadurch gekennzeichnet, dass der Zeolit vor seinem Einsatz mit wenigstens einem Kation aus der Gruppe der $Zn^{++}$, $Co^{++}$, $Mn^{++}$ und erdalkalischen Kationen substituiert wird.

11. Verfahren zur Herstellung von Jodbenzol, dadurch gekennzeichnet, dass man Benzol, Jod und Luft oder ein anderes sauerstoffhaltiges Gas mit einem Zeoliten von Typ ZSM—5, der mit wenigstens einem Kation aus der Gruppe der $Zn^{++}$, $Ca^{++}$ und $Mn^{++}$ Kationen substituiert wurde, bei einer Temperatur von 350 bis 450°C in Berührung bringt, wobei das Molekularverhältnis Luft/$I_2$ gleich 8 oder höher ist und die Raumgeschwindigkeit zwischen 1 und 100 kg/h Benzol pro kg Reinzeolit liegen sollte, während Jod in Form einer Benzollösung zugeführt wird, deren Konzentration zwischen 5 und 20 Gew./% liegt.

**Revendications**

1. Un procédé de synthèse d'iodobenzène par ioduration oxydative en phase gazeuse du benzène à l'aide d'iode et d'oxygène, d'air ou de tout autre gaz renfermant de l'oxygène, en présence d'un catalyseur zéolitique du groupe comprenant les zéolites du type ZSM—5 et du type ZSM—11, avec substition, avant l'utilisation, par au moins un cation métallique bivalent ou trivalent.

2. Un procédé selon la revendication 1, caractérisé en ce que l'ioduration est effectuée à une température comprise entre 200 et 550°C.

3. Un procédé selon la revendication 2, caractérisé en ce que la température de ioduration est comprise entre 350 et 450°C.

4. Un procédé selon la revendication 1, caractérisé en ce que l'agent d'oxydation est constitué par de l'air et en ce que le rapport molaire "air/$I_2$" est égal ou supérieur à la valeur du rapport stoechiométrique.

5. Un procédé selon la revendication 3, caractérisé en ce que le rapport molaire est supérieur à 10.

6. Un procédé selon la revendication 1, caractérisé en ce que la concentration en iode du benzène d'amenée est comprise entre 0,5 et 50%.

7. Un procédé selon la revendication 6, caractérisé en ce que la concentration en iode du benzène d'amenée est comprise entre 5 et 20% en poids.

8. Un procédé selon la revendication 1, caractérisé en ce que la vitesse spatiale est comprise entre 0,1 et 100 kg/h de benzène par kg de zéolite pur (à l'exclusion du liant).

9. Un procédé selon la revendication 8, caractérisé en ce que la vitesse spatiale est comprise entre 1 et 20 kg/h de benzène par kg de zeolite pur (à l'exclusion du liant).

10. Un procédé selon la revendication 6, caractérisé en ce qu'avant son utilisation, on soumet le zéolite à l'échange avec un moins un cation du groupe comprenant $Zn^{++}$, $Co^{++}$, $Mn^{++}$ et les cations alcalino-terreux.

11. Un procédé de synthèse d'iodobenzène, caractérisé en ce qu'on met en contact du benzène, de l'iode et de l'air, (ou un autre gaz renfermant de l'oxygène) et un zéolite du type ZSM—5 substitué par au moins un cation du groupe comprenant $Zn^{++}$, $Mn^{++}$ et $Ca^{++}$, à une température comprise entre 350° et 450°C, le rapport molaire "air/$I_2$" étant égal ou supérieur à 8, la vitesse spatiale étant comprise entre 1 et 100 kg/h de benzène par kg de zéolite pur, cependant que l'iode est amené sous la forme d'une solution benzénique d'une concentration comprise entre 5 et 20% en poids.